(19) **Europäisches Patentamt European Patent Office Office européen des brevets**

(11) **EP 3 124 079 A1**

(12) **DEMANDE DE BREVET EUROPEEN**

(43) Date de publication:
01.02.2017 Bulletin 2017/05

(51) Int Cl.:
*A61Q 5/00* (2006.01)          *A61Q 19/00* (2006.01)
*A61Q 19/08* (2006.01)          *A61K 8/97* (2017.01)

(21) Numéro de dépôt: **16181341.5**

(22) Date de dépôt: **26.07.2016**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats d'extension désignés:
**BA ME**
Etats de validation désignés:
**MA MD**

(30) Priorité: **28.07.2015 FR 1557195**

(71) Demandeur: **Laboratoires M & L
04100 Manosque (FR)**

(72) Inventeurs:
• **TOUREL, Cécile
13090 AIX EN PROVENCE (FR)**
• **MILLET, Magali
04190 LES MEES (FR)**

(74) Mandataire: **Novagraaf Technologies
Bâtiment O2
2, rue Sarah Bernhardt
CS90017
92665 Asnières-sur-Seine Cedex (FR)**

(54) **UTILISATION COSMÉTIQUE D'UNE PURÉE VÉGÉTALE**

(57)     La présente invention se rapporte à l'utilisation cosmétique d'une purée végétale, en particulier l'utilisation cosmétique d'une purée végétale avec une granulométrie moyenne de 50 μm à 2 cm, à un traitement cosmétique non thérapeutique comprenant l'application sur la peau et/ou les phanères de ladite purée végétale.

La présente invention se rapporte également à une purée végétale cosmétique obtenue par un procédé comprenant notamment les étapes de tamisage, broyage, éventuellement d'affinage, d'homogénéisation du végétal, suivi d'un traitement microbiologique du broyat constituant la purée végétale.

EP 3 124 079 A1

## Description

La présente invention se rapporte à l'utilisation cosmétique d'une purée végétale, en particulier l'utilisation cosmétique d'une purée végétale avec une granulométrie moyenne de 50 μm à 2 cm.

La présente invention se rapporte notamment à l'utilisation cosmétique d'une purée végétale avec une granulométrie moyenne de 50 μm à 2 cm et une teneur en matière sèche de 5 à 40 % en poids par rapport au poids total de la purée.

La présente invention se rapporte également à une purée végétale cosmétique obtenue par un procédé comprenant notamment les étapes de tamisage, broyage, éventuellement d'affinage, d'homogénéisation du végétal, suivi d'un traitement microbiologique du broyat affiné constituant la purée végétale.

La présente invention se rapporte également à un procédé de traitement cosmétique non thérapeutique comprenant l'application sur la peau et/ou les phanères d'une purée végétale avec une granulométrie moyenne de 50 μm à 2 cm.

La présente invention trouve une application notamment dans les domaines cosmétique et/ou dermatologique.

Dans la description ci-dessous, les références entre crochets ([ ]) renvoient à la liste des références présentée à la fin du texte.

## Etat de la technique

La peau est l'organe le plus lourd du corps humain et possède une architecture très complexe. Barrière entre les milieux extérieur et intérieur de notre corps, son fonctionnement a deux finalités : assurer la communication entre l'organisme et l'environnement extérieur et le protéger des agressions.

Les fonctions de la peau sont multiples, mais quatre principaux rôles sont reconnus : 1) protection car la peau constitue une barrière physique, immunologique et biologique, 2) thermorégulation à travers son rôle dans le maintien de la température corporelle, 3) métabolisme à travers l'hypoderme, lieu de stockage principal des triglycérides produits lors la lipogenèse et qui pourront être mobilisés pour la lipolyse et 4) sensation, car est à l'origine de l'un des cinq sens : le toucher. Le vieillissement cutané peut entraîner une altération de perception de l'image corporelle avec, à terme, des symptômes dysmorphophobiques ou dépressifs. Par ailleurs, les personnes ayant des signes de vieillissement cutané sont souvent perçues par notre société comme étant en mauvaise santé et incapables d'initiatives. Ainsi, la demande thérapeutique afin de prévenir le vieillissement cutané est aujourd'hui de plus en plus forte. Pour y répondre, une meilleure connaissance de sa physiopathologie, mais aussi de ses conséquences cliniques, est nécessaire. Comme tous les autres organes, la peau est soumise au vieillissement. Les facteurs chronologiques, génétiques et les facteurs endocriniens, par exemple les bouleversements post-ménopausiques chez la femme, déterminent le vieillissement cutané intrinsèque.

D'autre part, la fermeté cutanée diminue avec l'âge. La fermeté cutanée est principalement apportée par le compartiment dermique. Celui-ci est constitué de fibroblastes qui sécrètent, dégradent et remodèlent une matrice extra-cellulaire qui occupe plus de 90% de ce derme. Les composants principaux de cette matrice extra-cellulaire sont :

- Les collagènes, notamment les collagènes fibrillaires, de type I et III qui sont les collagènes fibrillaires majoritaires du derme et lui confèrent sa résistance et fermeté. Les collagènes fibrillaires de type IV et VII qui sont présents dans les lames basales séparant le derme des autres compartiments (épiderme, vaisseaux) et auxquelles ils apportent ancrage et solidité.
- Les fibres élastiques composées d'un polymère de tropoélastine aligné sur un support microfibrillaire composé majoritairement de fibrilline1. Les fibres élastiques assurent l'élasticité de la peau et son retour à l'état initial, sans déformation, après un étirement

Ces composants primordiaux pour le maintien des propriétés mécaniques de la peau, sont moins synthétisés au cours du vieillissement, et de plus en plus dégradés sous l'action de protéases matricielles comme les métalloprotéinases (MMPs) dont les collagènases, l'élastase. Il est donc important de relancer leur synthèse et leur fonctionnalité dans une peau adulte.

Il existe dans l'état de la technique de nombreuses compositions cosmétiques, notamment topiques, pour le traitement de la peau et/ou des cheveux. En particulier, il existe de nombreuses compositions comprenant différents principes actifs cosmétiques. Toutefois, ces compositions présentent des efficacités relatives par rapport au traitement cosmétique voire contestées dans certains cas par des associations de consommateurs.

L'efficacité relative des compositions disponibles dans l'état de la technique est notamment liée à la concentration faible de principes actifs dans les compositions cosmétiques. En particulier, lors de l'utilisation de produits/d'extraits issus de la nature, les compositions cosmétiques connues présentent de faibles concentrations en principe actif due notamment à la faible teneur en matière sèche présente dans les extraits.

Depuis plusieurs années, une majorité d'utilisateurs de produits cosmétiques et de consommateurs recherchent des produits/compositions cosmétiques naturels, c'est-à-dire dont les principes actifs proviennent notamment de plantes et/ou végétaux et/ou produits obtenus à partir de la nature, tel que le miel etc. En d'autres termes, les utilisateurs/consommateurs recherchent des produits cosmétiques présentant, notamment une haute naturalité.

Il existe dans l'état de la technique des compositions cosmétiques dont certains de leurs principes actifs et/ou ingrédients sont issus de la nature et/ou issus de plantes/végétaux, par exemple issus de l'agriculture biologique présentant ainsi une meilleure naturalité. Toutefois, la teneur en principes actifs dans ces compositions reste limitée.

De plus, les compositions cosmétiques à forte naturalité connues peuvent présenter des problèmes d'aspect et/ou de parfum. En effet, certaines cosmétiques dites très naturelles peuvent présenter un aspect visuel déplaisant, par exemple avec un déphasage, et/ou une odeur désagréable. Ces inconvénients sont des éléments qui dissuadent les utilisateurs/consommateurs quant à l'utilisation de ces produits naturels.

En outre, les compositions connues comprenant et/ou constituées des extraits bruts et/ou broyés de végétaux, par exemple des masques et/ou des broyats de végétaux, nécessitent après application une étape de rinçage, par exemple à l'eau, limitant ainsi leur utilisation, notamment pour un usage quotidien.

De plus, les compositions connues comprenant et/ou constituées des extraits bruts et/ou broyés de végétaux nécessitant après application une étape de rinçage limite/réduit le temps de contact entre la peau et la composition diminuant/réduisant ainsi automatiquement l'efficacité de ces compositions.

Il existe donc un réel besoin de trouver de nouvelles compositions palliant ces défauts, inconvénients et obstacles de l'art antérieur, en particulier des compositions cosmétiques à forte naturalité, présentant notamment un aspect visuel et/ou odeur adapté(s) aux applications cosmétiques.

Il existe en outre un réel besoin de trouver de nouvelles compositions comprenant de fortes concentrations en principes actifs naturels avec de préférence une faible teneur en ingrédients synthétiques, par exemple les ingrédients de formulation.

Il existe également un réel besoin de trouver de nouvelles compositions cosmétiques à forte naturalité présentant une bonne efficacité cosmétique, par exemple pour le traitement anti-âge permettant notamment de lisser la surface de la peau, raffermir la peau et/ou prévenir ou traiter le vieillissement cutané.

Description de l'invention

La présente invention a précisément pour but de répondre à ces besoins et inconvénients en proposant l'utilisation cosmétique d'une purée végétale ayant une granulométrie moyenne de 50 $\mu$m à 2 cm.

Les inventeurs de la présente sont en effet les tout premiers à avoir mis en évidence, de manière tout à fait inattendue, que l'utilisation d'une purée végétale ayant une granulométrie moyenne de 50 $\mu$m à 2 cm, en particulier une purée végétale avec une granulométrie moyenne de 50 $\mu$m à 2 cm et une teneur en matière sèche de 5 à 40 % en poids par rapport au poids total de la purée, présente avantageusement une concentration de principes actifs naturels très importante et permet avantageusement de :

- comprendre le totum du végétal/ de la plante dont elle est issue
- présenter une teneur en matière sèche élevée,
- conserver l'intégralité du végétal/plante dont elle est issue,
- conserver la coloration et les senteurs des végétaux/plantes dont elle est issue, et
- comprendre une forte teneur en micronutriments.

Ainsi, l'utilisation de la purée selon l'invention permet avantageusement de bénéficier de la majorité des principes actifs cosmétiques présents dans le végétal assurant à la fois une forte naturalité et une efficacité dans les traitements cosmétiques améliorés. En outre, l'utilisation de la purée selon l'invention permet de part la présence de la majorité des principes actifs présents dans le végétal dont elle est issue permet d'obtenir une forte concentration en principe actifs. En outre, la purée selon l'invention permet avantageusement d'obtenir une très bonne efficacité pour le traitement cosmétique dans laquelle elle est utilisée de part la présence de fortes concentrations en principes actifs.

En outre, les inventeurs ont démontré de manière surprenante que la purée végétale avec une granulométrie moyenne de 50 $\mu$m à 2 cm permet avantageusement son utilisation dans diverses galéniques cosmétiques, par exemple gel aqueux ou hydroalcoolique, gel huileux, émulsion, lait, lotion, eau, huile, savon, base moussante, une poudre, un sérum, pour divers types d'applications cosmétiques, par exemple soin non rincé pour visage et/ou corps et/ou cheveux, produits d'hygiène pour visage et/ou corps et/ou cheveux, masque, produit démaquillant, produit de maquillage.

De plus, les inventeurs ont démontré de manière surprenante que les compositions cosmétiques comprenant une purée végétale avec une granulométrie moyenne de 50 $\mu$m à 2 cm peuvent être appliquées directement sur la peau et ne nécessite pas d'étape de rinçage après application.

En d'autres termes, les inventeurs de part notamment la sélection particulière de la granulométrie de la purée, ont obtenu, de manière surprenante, des compositions cosmétiques comprenant une purée végétale ne nécessitant pas de rinçage, avantageusement l'utilisation cosmétique de purée végétale ne nécessitant pas de rinçage.

Dans la présente par « végétal » on entend tout végétal connu de l'homme du métier. Il peut s'agir par exemple de tout organisme eucaryote pluricellulaire du règne végétal. Il peut s'agir, par exemple d'eucaryotes pluricellulaires réalisant la photosynthèse. Il peut s'agir par exemple de végétal appartenant à la classe Magnoliopsida, par exemple les pivoines, les rosiers, le bigaradier, les framboisiers, le jasmin, la violette, le chèvrefeuille, l'argousier, la griotte, le grenadier, l'églantier, l'acérola, la carotte, le prunier, le melon, le manguier, le potiron. Il peut s'agir par exemple d'un végétal appartenant à la classe Liliopsida, par exemple le lys. Il peut s'agir également d'un végétal appartenant à la famille des Verbenaceae, par exemple la verveine. Il peut s'agir également d'agrumes, par exemple de citrons, de clémentines, de kumquats, de bergamotes, de limes, de limettes, de mandarines, d'oranges, de pamplemousses, de pomelos, de tangerines, de combavas, de yuzus et/ou de cédrats. Selon l'invention, le végétal peut être choisi, par exemple, dans le groupe comprenant les pivoines, les rosiers, le bigaradier, les framboisiers, les abricotiers, le murier, le jasmin, la violette, le safran, le chèvrefeuille, le lys, le sorbier, la verveine, l'argousier, la pêche, la mirabelle, les agrumes, la griotte, la cerise, la pomme, le cassis, la canneberge, le kiwi, la poire, la groseille, la fraise, la grenade, la myrtille, la mure, le sorbe, l'églantier, l'acérola, la carotte, la prune, le melon, la goyave, la mangue, le potiron ou un mélange de ceux-ci.

Il peut s'agir par exemple d'un végétal ou matière végétale frais ou surgelé. Par exemple il peut s'agir d'une purée obtenue à partir de tout ou partie d'un végétal frais ou surgelé.

Dans la présente par végétal frais ou matière végétale fraiche on entend un végétal ou une matière végétale fraîchement récoltée, par exemple récoltée moins de 48 heures préalablement à sa transformation en purée. Il peut s'agir par exemple d'un végétal ou une matière végétale n'ayant subi aucun traitement et/ou altération de nature physico-chimique, enzymatique, microbiologique, thermique. Il peut s'agir également d'un végétal ou une matière végétale tel que mentionné ci-dessus surgelée, par exemple d'un végétal ou une matière végétale fraiche dont la surgélation est avantageusement réalisée moins de 48 heures après récolte. La surgélation permet avantageusement de préserver les qualités organoleptiques du végétal ou de la matière végétale et de stopper la dégradation microbiologique et enzymatique du végétal ou de la matière végétale.

Dans la présente la purée peut être issue de tout ou partie du végétal. Il peut s'agir par exemple d'une purée issue de fruits, de fleurs, de tiges, de racines, de graines, de feuilles ou un mélange de ceux-ci. Il peut s'agir avantageusement d'une purée de fruits, de fleurs ou un mélange de celles-ci.

Dans la présente par « granulométrie moyenne » on entend la mesure qui correspond à la valeur, par exemple en microns, à laquelle 50% des particules de l'échantillon est inférieur à ladite valeur et 50% de des particules de l'échantillon est supérieur à ladite valeur. Cette valeur est également dénommé diamètre médian en masse (« Mass médian Diameter » ou MMD).

Dans la présente la granulométrie moyenne peut être mesurée par tout procédé connu de l'homme du métier et/ou tout dispositif connu de l'homme du métier. Il peut s'agir par exemple d'un dispositif disponible dans le commerce, par exemple le Mastersize 2000.

Selon l'invention la granulométrie moyenne de la purée végétale peut être comprise de 50 $\mu$m à 2 cm, par exemple de 50 $\mu$m à 150 $\mu$m, de 200 $\mu$m à 500 $\mu$m, de 500 $\mu$m à 1 mm, de 1 mm à 1 cm.

Avantageusement, lorsque la purée a une granulométrie moyenne de 200 $\mu$m à 500 $\mu$m, elle est particulièrement adaptée pour une utilisation comme produit gommant, notamment un gommant pour le visage.

Avantageusement, lorsque la purée a une granulométrie moyenne de 500 $\mu$m à 1 mm, elle est particulièrement adaptée pour une utilisation comme produit gommant, notamment un gommant pour le corps.

Avantageusement, lorsque la purée a une granulométrie moyenne de 1 mm à 1 cm, elle est particulièrement adaptée pour une utilisation comme masque, notamment un masque visage.

Selon l'invention, le pH de la purée végétale peut être compris de 1 à 6, par exemple de 3 à 5, de 4 à 5, égale à environ 3,5.

Selon l'invention, la purée végétale peut comprendre une teneur en matière sèche supérieure à 5% en poids, de 5 à 40 % en poids, de 10 à 35% en poids, préférentiellement de 10 à 25% en poids par rapport au poids total de la purée.

Dans la présente, la mesure de la teneur en matière sèche peut être réalisée par tout procédé et/ou dispositif connu de l'homme du métier. Il peut s'agir par exemple d'un procédé utilisant un dessiccateur, par exemple un dessiccateur halogéné. Il peut s'agir également d'un procédé comprenant, à partir d'un échantillon, par exemple 5 g de purée végétale, le chauffage dudit échantillon jusqu'à une température de 105°C et mesure du poids en fonction du temps, la mesure étant terminée dès que le poids mesuré est stable pendant au moins 2 minutes. La teneur en matière sèche correspondant au rapport du poids final mesuré sur le poids initial multiplié par 100.

Selon l'invention, la purée végétale utilisée peut être obtenue à partir d'un procédé particulier, il peut s'agir par exemple d'un procédé comprenant les étapes suivantes :

a) broyage du végétal,

b) traitement microbiologique choisi parmi un traitement thermique du broyat obtenu à l'étape a) à une température de 20 à 150°C, un traitement haute pression à une pression de 2000 à 5000 bars, par un traitement par ionisation, un traitement par application d'hautes fréquences, un traitement par application d'un courant ohmique, un traitement par pasteurisation en autoclave, ou par des procédés de stérilisation tels que l'UHT, par infusion ou d'injection directe de vapeur dans le broyat, ou un mélange de ceux-ci, ledit broyat traité constituant la purée végétale.

Dans la présente, le végétal utilisé à l'étape a) peut être tout végétal tel que défini ci-dessus.

Avantageusement, lorsque le végétal est un mélange de fruits et de fleurs, le procédé ne comprend pas d'étape éventuelle d'ajout d'eau. En particulier, les inventeurs ont démontré de manière surprenante, notamment lorsque le végétal est un mélange de fruits et de fleurs, que le procédé de l'invention permet l'obtention d'une purée avec une granulométrie fine, sans ajout additionnel d'eau permettant avantageusement de faciliter l'obtention de la purée, d'augmenter considérablement la teneur en matière sèche.

Dans la présente, l'étape a) de broyage peut être réalisée par tout moyen connu de l'homme du métier. Il peut s'agir par exemple d'un procédé utilisant un broyeur disponible dans le commerce, par exemple un broyeur Stephan.

Dans la présente, l'étape a) de broyage peut être réalisée avec un broyeur rotatif, par exemple à une vitesse de 2500 à 3500 tours par minute, par exemple à une vitesse de 3000 tours par minute.

Selon l'invention, l'étape a) de broyage peut être réalisée pendant un temps adapté afin d'obtenir un broyat susceptible d'être traité.

Dans la présente, l'étape a) de broyage peut être mise en oeuvre pendant un temps compris de 1 à 15 minutes, par exemple de 5 à 15 minutes. L'homme du métier de par ses connaissances générales peut déterminer/adapter le temps de broyage adapté, par exemple en fonction du végétal.

L'étape a) de broyage peut également être réalisée à une température comprise de -2 à 5°C, par exemple de -2 à 2°C.

Alternativement, l'étape a) de broyage peut comprendre simultanément une étape de chauffage du végétal et/ou du broyat permettant avantageusement l'obtention d'un broyat à une température comprise de -2°C à 30°C, de -2°C à 25°C, par exemple de 5 à 15°C.

Dans la présente, l'étape a) de broyage peut comprendre une deuxième étape de broyage a2) correspondant à une étape d'affinage. Dans la présente, l'étape d'affinage a2) permet avantageusement de réduire la taille des particules végétales.

Dans la présente, l'étape a2) d'affinage peut être réalisée par tout procédé connu de l'homme du métier. Il peut s'agir par exemple d'une étape d'affinage par broyage. Dans la présente, l'étape a2) d'affinage peut également être mise en oeuvre par tout moyen connu de l'homme du métier. Il peut s'agir par exemple d'un procédé utilisant un dispositif de type affineuse ou Comitrol. Il peut s'agir par exemple d'une affineuse disponible dans le commerce, par exemple une affineuse de marque Innotec.

Avantageusement, l'étape d'affinage permet d'obtenir une granulométrie moyenne dans le broyat inférieur à 2 mm, par exemple entre 200 et 500$\mu$m, de préférence inférieure à 100$\mu$m, avantageusement inférieure à 50 $\mu$m.

Avantageusement, l'étape d'affinage permet une réduction dimensionnelle des particules de végétaux, réduction qui est avantageusement supérieure à celle apportée par l'étape a) de broyage.

Dans la présente, lorsque le végétal utilisé dans le procédé précité est un végétal surgelé, le procédé peut comprendre une étape préalable a0) de décongélation du végétal surgelé.

Dans la présente, l'étape préalable a0) de décongélation peut être réalisée par tout procédé dispositif connu de l'homme du métier. Il peut s'agir par exemple d'un chauffage, par exemple par microonde, d'une mise à température, par exemple en déposant le végétal surgelé dans une enceinte en froid positif, par exemple dans une chambre froide à une température de 0 à 5°C.

Dans la présente, l'étape préalable a0) peut être mise en oeuvre pendant un temps de 1 à 48 heures, par exemple de 5 à 32 heures, de 10 à 28 heures, égal à 24 heures.

Avantageusement, l'étape préalable a0) est mise en oeuvre pendant un temps suffisant afin d'obtenir un végétal à une température comprise de -2°C à 5°C. Avantageusement, lorsque le végétal est à une température positive de 2 à 5°C, cela permet de faciliter le broyage et d'obtenir un broyat à l'issu de l'étape a) moins épais.

Dans la présente, l'étape b) de traitement de l'homogénat peut être un traitement microbiologique permettant avantageusement de diminuer la charge microbienne du broyat affiné. Il peut s'agir de tout traitement connu de l'homme du métier, par exemple un traitement par ionisation, micro-ondes, par application d'hautes fréquences, par application d'un courant ohmique, par application de hautes pressions, de pasteurisation en autoclave, ou par des procédés de stérilisation, par exemple des procédés de stérilisation Upérisation à Haute Température (UHT), par infusion ou d'injection directe de vapeur dans le broyat, ou un mélange de ceux-ci.

Dans la présente, l'étape b) peut comprendre :

une première étape b') de traitement en flux discontinu comprenant, par exemple,

- une étape de chauffage du broyat affiné à une température comprise de 80 à 100°C, par exemple de 85 à 95°C, et
- maintien de la température pendant un temps de 2 à 30 minutes, et
- une étape de refroidissement pour atteindre la température ambiante pendant un temps de 10 à 30 min

ou une première étape b") traitement en flux continu comprenant, par exemple,

- une étape de chauffage du broyat affiné à une température de 80 à 150°C,
- maintien de la température pendant un temps de 1 à 3 minutes, et
- une étape de refroidissement pour atteindre la température ambiante pendant un temps de 1 à 5 min.

Dans la présente, l'éventuelle étape b') de traitement en flux discontinu de chauffage et de maintien de la température peut être réalisé par tout dispositif de chauffage connu de l'homme du métier et/ou disponible dans le commerce. Il peut s'agir par exemple d'un autoclave, par exemple un autoclave commercialisé par la société Lagarde.

Dans la présente, l'éventuelle étape b") de traitement en flux continu le chauffage peut être réalisée par tout dispositif de chauffage connu de l'homme du métier et/ou disponible dans le commerce. Il peut s'agir par exemple d'un chauffage Ohmique, par exemple une ligne pilote à chauffage ohmique d'une capacité de 200 kg/h et d'une puissance de 2X 30 kW.

Dans la présente, l'éventuelle étape b") de traitement en flux continu le refroidissement peut être réalisé par tout procédé/dispositif connu de l'homme du métier. Il peut s'agir par exemple d'un système de refroidissement par échangeur tubulaire simple.

Dans la présente, le procédé peut comprendre en outre une étape i) de désaération du végétal antérieure à l'étape b) de traitement.

Dans la présente, l'étape i) de désaération peut être réalisée sous un vide compris de - 1,5 x $10^5$ Pascal (-1,5 bars) à - 0,5 x $10^5$ Pascal (-0,5 bars), de préférence de - 0,9 x $10^5$ Pascal (-0,9 bars).

Dans la présente, l'étape i) de désaération peut être réalisée à une température comprise de 20 à 55 °C, par exemple de 40 à 50 °C.

Dans la présente, l'étape i) de désaération peut être mise en oeuvre par tout procédé de désaération connue de l'homme du métier. Il peut s'agir par exemple du procédé comprenant une projection, sous vide, d'une composition sur un disque en rotation provoquant, par exemple une dispersion du produit en gouttelettes, l'air étant supprimé par le vide.

Dans la présente, l'étape i) de désaération peut être mise en oeuvre par tout dispositif de désaération connue de l'homme du métier. Il peut s'agir par exemple d'un dispositif disponible dans le commerce, par exemple un désaérateur avec une capacité de 40 litres, il peut s'agir par exemple d'un désaérateur commercialisé sous la marque Fryma modèleLVE/B.

Selon l'invention, le procédé peut comprendre en outre une étape ii) d'homogénéisation postérieure à l'étape a) et/ou i). Il peut s'agir par exemple d'une homogénéisation par application de pression et peut être réalisée par tout moyen connu de l'homme du métier. Il peut s'agir par exemple d'une homogénéisation par application de pression utilisant un dispositif disponible dans le commerce, par exemple un homogénéisateur Molecola commercialisé par la société Bertoli.

Dans la présente, la pression appliquée à l'étape ii) peut être comprise de 100 à 500 bars, par exemple de 300 à 500 bars.

Dans la présente, la pression appliquée à l'étape ii) peut comprendre une première application de pression, par exemple entre 325 et 375 bars, puis une seconde application de pression, par exemple entre 375 et 425 bars.

Dans la présente, lors de l'application de la pression à l'étape ii), le débit de passage du broyat affiné peut être de 60 à 90 kg de broyat affiné par heure, par exemple égal à 80 kg/h.

Dans la présente, le procédé peut comprendre en outre, préalablement ou après l'étape a) de broyage une étape iii) de tamisage du broyat et/ou du broyat affiné.

Dans la présente, l'étape iii) peut être réalisée par tout moyen connu de l'homme du métier. Il peut s'agir par exemple d'un tamisage à travers un tamis comprenant des pores de de 0,5 à 3 millimètres de diamètre, de 18/10ème à 6/10ème de millimètres de diamètre, par exemple de 15/10ème à 8/10ème de mm de diamètre.

Dans la présente, l'étape iii) peut être réalisée par un dispositif disponible dans le commerce, il peut s'agir d'une raffineuse horizontale de la marque Rossi Catelli.

Dans la présente, l'étape iii) peut être réalisée à une température comprise entre 5 et 35°C, par exemple entre 10 et 25°C.

La présente invention a également pour objet une purée végétale susceptible d'être obtenue par le procédé ci-dessus.

Avantageusement, les inventeurs ont démontré que la purée végétale obtenue par le procédé ci-dessus présente de fortes teneurs en molécules actives du végétal dont elle est issue tout en présentant des propriétés organoleptiques et une stabilité microbiologique adaptée pour son utilisation cosmétique. En outre, les inventeurs ont démontré de manière surprenante que la purée de végétal obtenue par le procédé ci-dessus présente une homogénéisation, une stabilité microbiologique adaptée à son utilisation cosmétique tout en présentant une forte teneur en molécules issues du végétal permettant avantageusement une efficacité cosmétique renforcée.

Les inventeurs ont également démontré que la purée de végétal obtenue par le procédé comprend avantageusement le totum du végétal dont elle est issue. En particulier, la purée de végétal comprend avantageusement :

- les macromolécules constitutives des fleurs et fruits, par exemple la cellulose, les pectines, les lignines, les pigments, les pollens pour les fleurs, ainsi que les contenus intracellulaires, par exemple les acides nucléiques, les lipides, les anthocyanes, SM,
- les métabolites primaires, par exemple les acides aminés, les vitamines, les nucléotides, les antioxydants, les acides organiques, les polyols, et
- métabolites secondaires, par exemple les polyphénols, les terpénoïdes, les alcaloïdes.

En d'autres termes, la purée de végétal utilisée selon l'invention et/ou obtenus selon le procédé précité comprend avantageusement l'intégralité des constituants du végétal dont elle est issue à l'exception, par exemple des éléments susceptibles d'être éliminés à l'étape b) du procédé précité.

En outre, les inventeurs ont démontré de manière surprenante que la purée végétale avec une granulométrie moyenne de 50 $\mu$m à 2 cm permet avantageusement son utilisation dans diverses galéniques cosmétiques, par exemple gel aqueux ou hydroalcoolique, gel huileux, émulsion, lait, lotion, eau, huile, savon, base moussante, une poudre, un sérum, pour divers types d'applications cosmétiques, par exemple soin non rincé pour visage et/ou corps et/ou cheveux, produits d'hygiène pour visage et/ou corps et/ou cheveux, masque, produit démaquillant, produit de maquillage.

De plus, les inventeurs ont démontré que la purée végétale permet avantageusement de conserver les propriétés visuelles et olfactives du végétal dont elle est issue.

En outre, les inventeurs ont démontré de manière surprenante que la purée végétale selon l'invention et/ou obtenue selon le procédé précité comprend avantageusement une teneur en matière sèche de 5 à 40 % en poids par rapport au poids total de la purée permettant notamment de comprendre de nombreux principes actifs du végétal dont elle est issue.

De plus, les inventeurs ont démontré de manière surprenante que la purée obtenue, notamment lors de son utilisation cosmétique, par exemple lors ou après application sur la peau, ne nécessite pas d'étape de rinçage. L'absence de rinçage après application permet avantageusement d'augmenter le temps de contact sur la peau et avantageusement l'efficacité de la composition.

La présente invention a également pour objet l'utilisation d'une composition cosmétique comprenant une purée végétale telle que définie ci-dessus.

Selon l'invention, la purée végétale peut être présente à une concentration de 2 à 50 %, par exemple de 5 à 35%, de 10 à 30% en poids par rapport au poids total de la composition cosmétique.

Dans la présente par composition cosmétique on entend toute composition cosmétique connue de l'homme du métier. Il peut s'agir par exemple d'une composition topique, une composition capillaire. La composition cosmétique ou dermatologique peut être, par exemple une composition pour le soin du visage, du corps, des cheveux, par exemple des compositions pour le visage et/ou le corps et/ou les cheveux. La composition cosmétique ou dermatologique peut comprendre un ou plusieurs adjuvants connus de l'homme du métier. Il peut s'agir par exemple d'un ou plusieurs adjuvant(s) choisi(s) parmi des agents de type esters, des agents hydratants, des agents émollients, des agents épaississants minéraux, des agents épaississants organiques, associatifs ou non, des composés siliconés, des parfums, des conservateurs, des céramides, et pseudo-céramides, des vitamines et les provitamines, des protéines, des agents séquestrants, des agents alcanisants, des agents acidifiants, des agents anti-oxydants, des charges minérales, des colorants, des émulsionnants ou tout autre adjuvant pouvant être cité dans le dictionnaire INCI (International Nomenclature of Cosmetic Ingredients) publié par le PCPC (Personal Care Products Council).

Dans la présente, la composition cosmétique peut, par exemple, se présenter sous toute forme connue de l'homme du métier dans les domaines de la cosmétique et/ou de la dermatologie, sans aucune restriction galénique particulière. Il peut s'agir, par exemple d'une émulsion huile-dans-l'eau, eau-dans-l'huile, eau dans silicone, d'une émulsion multiple, d'une microémulsion, d'une nano-émulsion, d'une émulsion solide, d'un gel, d'un gel aqueux ou hydroalcoolique ou anhydre, d'une crème, d'un lait, d'une lotion, d'une pommade, d'un sérum, d'un baume, d'un onguent, d'un masque, d'une poudre, d'un support imbibé, par exemple un patch transdermique, d'une lotion aqueuse ou hydroalcoolique et/ou une cire, d'un produit de maquillage, par exemple un fond de teint, d'un shampooing, d'un après-shampooing, d'un masque, sérum, lotion capillaire, une eau.

Dans la présente, la composition cosmétique peut être obtenue par tout procédé approprié connu de l'homme du métier pour la fabrication d'une composition cosmétique. Il peut s'agir, par exemple d'un simple mélange. Il peut s'agir d'un mélange de matières tensioactives dans de l'eau. Il peut s'agir également, par exemple, d'un procédé comprenant une étape d'incorporation d'une phase interne dans une phase externe au moyen d'un émulseur, par exemple d'une turbine de type rotor-stator. Il peut s'agir également par exemple d'un procédé utilisant la Température d'Inversion de Phase (TIP), d'un procédé classiquement utilisé par l'homme de l'art pour obtenir des émulsions huile dans eau dont les gouttelettes dispersées sont particulièrement fines, par exemple avec un diamètre de 0,1 à 1 μm.

La présente invention a également pour objet un procédé de traitement cosmétique non thérapeutique comprenant une étape d'application sur la peau et/ou les phanères d'une purée végétale et/ou d'une composition cosmétique comprenant une purée végétale telle que définie ci-dessus.

Pour cette application, on peut, par exemple, utiliser les formes galéniques décrites ci-dessus. L'application sur la peau ou les cheveux peut être réalisée en fonction de la forme galénique utilisée.

Il peut s'agir, par exemple d'une simple application sur la peau ou d'une application accompagnée d'un massage.

Il peut s'agir, par exemple d'une simple application sur les cheveux ou d'une application suivi d'un rinçage.

L'application est de préférence réalisée avec une quantité suffisante de la composition afin que toute la surface de la peau ou du cheveu à traiter soit traitée. Il peut s'agir par exemple d'une application classique, Il peut s'agir par exemple d'une application classique, comme une crème sur la peau. Il peut s'agir par exemple aussi d'une application pour former un masque traitant.

L'application peut être toute application souhaitée par l'utilisateur/l'utilisatrice, par exemple une application quotidienne, biquotidienne, hebdomadaire et/ou bi-mensuelle. Il peut s'agir par exemple d'une application une fois par semaine, deux fois par semaine, trois fois par semaine ou plus, une fois par jour, deux fois par jour ou plus.

La présente invention se rapporte également à une trousse de soin cosmétique comprenant une purée végétale et/ou composition cosmétique comprenant une purée végétale selon la présente invention et un manuel ou une notice d'utilisation ou un support comportant des instructions pour l'emploi de la composition sur la peau. La trousse peut être par exemple un conditionnement.

Le manuel ou la notice peut avoir notamment pour fonction d'expliquer à l'utilisateur la manière et la fréquence d'application sur la peau de la composition de la présente invention.

Selon l'invention, la trousse de soin cosmétique peut également comprendre un applicateur, par exemple une spatule, une brosse, un applicateur dynamique, par exemple un dispositif de massage imprégné de la composition cosmétique de l'invention.

D'autres caractéristiques et avantages apparaîtront encore à l'homme du métier à la lecture des exemples ci-dessous, donnés à titre illustratif et non limitatif.

## EXEMPLES

### Exemple 1 : Exemple d'obtention d'une purée végétale

Dans le présent exemple, des purées ont été obtenues à partir de de roses et de framboises

Pour chaque batch 10kg de fleurs et 10kg de fruits préalablement décongelés stockées à 4°C depuis 12h00, ont été broyés avec un broyeur de marque Stephan environ 2 minutes en chauffant à une température comprise entre 10 et 15°C.

Le broyat a été ensuite raffiné à l'aide d'une raffineuse horizontale de marque Rossi Catelli.

Tableau 1 : caractéristiques de rendement et du procédé d'obtention de la purée

|  | Batch n°1 | Batch n°2 | Batch n°3 |
|---|---|---|---|
| Température de la mêlée après broyage en °C | 10,5 | 10,5 | 9,1 |
| Poids de la mêlée avant raffinage en kg | 18,24 | 20,98 | 20,02 |
| Poids de produit récupéré après raffinage en kg | 44,48 | | |
| Poids des déchets de raffinage en kg | 12,68 | | |
| Rendement en % | 77,8% | | |
| Température du produit après raffinage en °C | 25 | | |

Après raffinage, une première purée a été obtenue et a été homogénéisée dans un homogénéisateur Molecola Bertoli par application de pression à environ 400 bars. Une mesure du poids a été réalisée et le calcul du rendement de production effectué et était égal à 64,2%.

La purée a été ensuite conditionnée dans différentes barquettes de 400g et operculées. Les barquettes obtenues ont été ensuite pasteurisées pour conservation Les barquettes ont été pasteurisées à une température de 90°C jusqu'à obtenir une température à coeur de 85°C. Après pasteurisation, les barquettes ont été laissées refroidies jusqu'à obtenir une température de 25°C. Les paramètres de stérilisation sont décrits dans le tableau ci-dessous :

Tableau 2 : paramètres de stérilisation

|  | Barème pasteurisation : Cycle 85°C |
|---|---|
| Durée de montée en température | 15 minutes |
| Durée de pallier | 8 minutes |
| Température produit en fin de pallier | 86,4°C |
| Durée de refroidissement | 25 minutes |
| Température produit en fin de cycle | 25°C |
| Valeur pasteurisatrice | 0,896 |
| Durée totale de cycle | 48 minutes |

Les barquettes de purées pasteurisées ont été ensuite stockées à une température de -18°C.

Des analyses microbiologiques et qualitatives concernant l'aspect de la purée obtenue ont été ensuite réalisées. Pour l'analyse microbiologique le procédé mis en oeuvre correspond pour chacun des microorganismes au procédé décrit dans le tableau 3 ci-dessous.

| Microorganismes | Procédé |
|---|---|
| Escherichia Coli Betat glucosidase positive | Procédé Pétrifilm Select E.coli 3M-01/8-06/01 |
| Staphylocoques coagulase | Procédé NF V08-057-1 (janvier 2004) |
| Pseudomonas spp. Presomptifs | Procédé NF EN IS 13270 (novembre 2010) |
| Levures | Procédé NF V08-059 (novembre 2002) |
| Moisissures | Procédé NF V08-059 (novembre 2002) |
| Microorganismes 30°C | Procédé Pétrifilm flore totale 3M-01/1-09/89 |

Il s'agissait en particulier de la détermination du nombre d'unité formant colonie par gramme.

Concernant l'analyse de l'aspect, le procédé mis en oeuvre correspond à l'observation visuelle de la purée, notamment d'un éventuel changement de la couleur de la purée. En outre une mesure de L, a et b correspondant à trois grandeurs caractérisant les couleurs, la clarté L et deux paramètres a et b, qui expriment l'écart de la couleur par rapport à celle d'une surface grise de même clarté. La composante L* est la clarté, qui va de 0 (noir) à 100 (blanc). La composante a* représente une gamme de 600 niveaux sur un axe rouge (+299 valeur positive) → vert (-300 valeur négative) en passant par le gris (0). La composante b* représente une gamme de 600 niveaux sur un axe jaune (+299 valeur positive) → bleu (-300 valeur négative) en passant par le gris (0). La mesure de L, a et b a été effectuée avec un appareil de mesure de couleur CIELab PCE-TCD 100. Il s'agissait en particulier de la détermination de l'aspect de la purée avant et après pasteurisation.

Une détermination de la quantité de différentes molécules présentes dans la composition a été effectuée, il s'agissait en particulier de la détermination de la concentration en sucres, en acides aminés, en acide ascorbique, en acide déhydro-ascorbique, en vitamine C totale, en polyphénols totaux, en matière sèche, en antho-cyanes totales, en caroténoïdes totaux, en glucose, en fructose, en saccharose, en sucres et ORAC.

Pour les acides aminés totaux, le procédé était un procédé à la ninhydrine comme suit :

Peser 0,8g de Ninhydrine dans un pilulier, ajouter 80 ml d'éthanol et 10 ml d'acide acétique glacial sous la hotte. Mettre aux ultrasons pendant 10 minutes.

Préparation des standards : Calibration Leucine (20-400 ppm)
Solvant de dilution : Eau distillée
Préparation de la Solution Mère : Peser exactement mLEU = 0,04g de Leucine (LEU) dans un tube à vis et compléter jusqu'à exactement VF = 20g avec de l'eau distillée (SM = 0,2%). Passer le tube aux ultrasons jusqu'à dissolution complète de la leucine (≈5').

Calculer la concentration exacte de la SM :

$$C_{SM}(ppm) = \frac{100 \times m_{LEU}}{V_F} \times 10000$$

avec mLEU en g et VF en g

La préparation des standards a été réalisée comme décrit dans le tableau 4 ci-dessous :

Tableau 4 : caractéristiques des standards

| N°Standard | Solution de prélèvement | Volume prélevé $V_1$ (g) | Volume final $V_2$(g) | Concentration en LEU (ppm) |
|---|---|---|---|---|
| Std 5 | SM | 4 | 20 | 400 |
| Std 4 | SM | 2 | 20 | 200 |
| Std 3 | SM | 1 | 20 | 100 |
| Std 2 | SM | 0,5 | 20 | 50 |
| Std 1 | Std 4 | 1 | 10 | 20 |

Le calcul des concentrations exactes en LEU a été réalisé par :

$$C_{Std}(ppm) = \frac{C_{SM} \times V_1}{V_2}$$

avec CSM en ppm, et $V_1$ et $V_2$ en g

La préparation des échantillons a été réalisée par filtration des extraits à analyser, dilution en pesant dans un tube à vis exactement 1g d'extrait filtré et compléter jusqu'à 5g avec de l'eau distillée.

Pour chaque échantillon, les mesures ont été effectuées trois fois et sont décrites dans le tableau 5 ci-dessous.

Tableau 5 : conditions de mesures

| | CALIBRATION | BLANC CALIBRATION (Cuve 1) | ESSAIS | BLANC ECHANTILL ON (Cuve 1) |
|---|---|---|---|---|
| Volume Standards | 1 ml | - | - | - |
| Volume Extrait (1/5) | - | - | 1 ml | 1 ml |
| Volume H$_2$O | - | 1 ml | - | 2ml |
| Réactif Ninhydrine | 2 ml | 2 ml | 2 ml | - |
| FERMER LES TUBES AVEC UN BOUCHON PLASTIQUE | | | | |
| INCUBATION 5 minutes à 84°C | | | | |
| REFROIDISSEMENT SUR GLACE PENDANT 5 minutes | | | | |
| LECTURE AVEC UN SPECTROMETRE A UNE LONGUEUR D'ONDE DE $\lambda$ = 570 nm avec le BLANC en cuve 1 | | | | |

Le dosage des Sucres totaux a été réalisé par le procédé décrit ci-dessous. Les réactifs utilisés étaient une solution de phénol à 5% dans de l'eau, 2,5 g de cristaux de phénol ont été pesés directement dans une fiole jaugée de 50 ml et compléter jusqu'au trait de jauge avec de l'eau distillée puis passés aux ultrasons 10 minutes et complétés à nouveau avec de l'eau si nécessaire. De l'acide sulfurique concentré H$_2$SO$_4$ a été également utilisé. Pour la préparation des standards, une calibration Glucose (10-200 ppm) a été effectuée. Le solvant de dilution était de l'eau distillée, la préparation de la Solution Mère a été effectuée via le pesage exact de mGlc = 0,04g de Glucose (Glc) dans un tube à vis et complété jusqu'à exactement VF = 20g avec de l'eau distillée (SM = 0,2%). La concentration exacte calculée par :

$$C_{SM}(ppm) = \frac{100 \times m_{Glc}}{V_F} \times 10000$$

avec mAG en g et et VF en g

Les standards ont été préparés selon le tableau 6 ci-dessous.

Tableau 6 : caractéristiques des standards

| N°Standard | Solution de prélèvement | Volume prélevé V$_1$ (g) | Volume final V$_2$(g) | Concentration en Glc (ppm) |
|---|---|---|---|---|
| Std 5 | SM | 2 | 20 | 200 |
| Std 4 | SM | 1 | 20 | 100 |
| Std 3 | SM | 0,5 | 20 | 50 |
| Std 2 | Std 5 | 1 | 10 | 20 |
| Std 1 | Std 4 | 1 | 10 | 10 |

La préparation des échantillons a été réalisée via filtration des extraits à analyser, dilution en pesant dans un tube à vis exactement 0,1g d'extrait filtré et complétée jusqu'à 10g (voir 20g si l'extrait est riche en sucres) avec de l'eau distillée.

Pour chaque échantillon, les mesures ont été effectuées trois fois et sont décrites dans le tableau 7 ci-dessous.

Tableau 7 : procédé de mesure des sucres

| | CALIBRATION | ESSAIS | BLANC (cuve 1) |
|---|---|---|---|
| Volume Standards | 0,5 ml | - | - |
| Volume Extrait (1/100 ou 1/200) | - | 0,5 ml | - |
| Volume H$_2$O | - | - | 0,5ml |
| Solution phénol 5% | 0,5 ml | 0,5 ml | 0,5 ml |
| VORTEX | | | |
| H$_2$SO$_4$ c | 2,5 ml | 2,5 ml | 2,5 ml |
| VORTEX - INCUBATION 30' 40°C | | | |
| RETOUR TA (10' OBSCURITE) | | | |
| LECTURE AVEC UN SPECTROMETRE CALIBRE A UNE LONGUEUR | | | |
| D'ONDE DE λ = 485 nm avec le BLANC en cuve 1 | | | |

Les résultats obtenus sont exprimés en ppm.

Pour la détermination de la quantité d'acide ascorbique, d'acide déhydroascorbique et de vitamine C, le procédé mis en oeuvre était le procédé décrit dans Lykkesfeldt et al. Anal Biochem. 2000 Jun 15;282(1):89-93 en HPLC.

Pour la détermination de la quantité de polyphénols totaux en équivalent acide gallique, le procédé mis en oeuvre était le procédé décrit dans Georgé J Agric Food Chem. 2005 Mar 9;53(5):1370-3.

Pour la détermination de la quantité de matière sèche, le procédé mis en oeuvre était le procédé décrit dans l'Arrêté du 8 septembre 1977 relatif aux méthodes officielles d'analyse des produits diététiques et de régime.

Pour la détermination de la quantité d'anthocyanes totale, le procédé utilisé était le procédé décrit dans Guisti et al. 2001.

Pour la détermination de la quantité de glucose, Fructose, Saccharose, Sucre et ORAC, elle a été déterminée en utilisant un kit enzymatique disponible dans le commerce et/ou par spectroscopie.

Enfin, la détermination de la quantité de Caroténoïdes totaux a été effectuée par spectroscopie.

Les résultats sont présentés dans les tableaux 8 à 11 ci-dessous.

Tableau 8 : Analyses microbiologiques des produits :

| Nombre d'échantillon | Conditionnement | FAM (ufc/gr) | L/M (ufc/gr) |
|---|---|---|---|
| 1 | Barquette autoclavable congelé | 100 | <10 |

Tableau 9 : aspect visuelle des purées:

| | | Produit avant pasteurisation | Produit après pasteurisation à 85°C |
|---|---|---|---|
| Mesure de couleur | L | 25,85 | 26,12 |
| | a | 23,06 | 22,71 |
| | b | 4,12 | 4,20 |

Tableau 10 : teneurs en sucres en et acides aminés libres dans les purées obtenues

| DENOMINATION ECHANTILLON | SUCRES TOTAUX (ppm; Glucose Equivalent) | | ACIDES AMINES LIBRES TOTAUX (ppm; Leucine Equivalent) | |
|---|---|---|---|---|
| | Moyenne | Ecart-type | Moyenne | Ecart-type |
| Purée fleur/fruit 50/50 Sans traitement Raf 12/10ème - Homogénéisation 400 bars | 37445,95 | 768,49 | 3431,01 | 61,43 |
| Purée fleur/fruit 50/50 Autoclave 85°C à coeur Raf 12/10ème - Homogénéisation 400 bars | 33956,40 | 165,25 | 3158,55 | 24,52 |

Tableau 11 : teneurs d'exemple d'actifs cosmétiques dans les purées obtenues

| | Acide ascorbique (mg/100g) | Acide déhydro-ascorbique (mg/100g) | Vitamine C totale (mg/100 g) | Polyphénols totaux en équivalent acide gallique (mg/100g) | Matière sèche (g/100g) | Anthocyanes totales (mg/100 g) |
|---|---|---|---|---|---|---|
| Purée fleur/ fruit | <LQ | 15,0 | 15,0 | 265,2 | 9,7 | 222,0 |
| | Caroténoïd es totaux (mg/100g) | Glucose (g/100g) | Fructose (g/100g) | Saccharos e (g/100g) | Sucres (g/100g) | ORAC (mmol équivale nt trolox/10 0g) |
| Purée Fleur/ fruit | 375,6 | 1,63 | 2,06 | Abs | 3,69 | 9,2 |

Ces résultats démontrent clairement que la purée obtenue est conforme aux critères microbiologiques et physico-chimiques requis pour permettre une application dans une composition cosmétique. Par ailleurs, la purée obtenue comprend avantageusement et de manière surprenante des teneurs en molécules identiques à celle du végétal dont elle est issue, elles ont donc été préservées et sont avantageusement présentes dans des quantités requises pour permettre une activité sur la peau.

**Exemple 2 : Exemple de compositions cosmétiques comprenant** une purée **végétale**

Le tableau 12 ci-dessous résume les ingrédients utilisés et présente des exemples de compositions cosmétiques comprenant des purées végétales.
La purée végétale utilisée correspond à la purée obtenue dans l'exemple 1 précité.

Tableau 12 : ingrédients pour la fabrication de compositions cosmétiques

| Phases | INGREDIENTS | Composition 1 (Pourcentage en poids par rapport au poids total de la composition) | Composition 2 (Pourcentage en poids par rapport au poids total de la composition) |
|---|---|---|---|
| A | Eau | QSP 100 | QSP 100 |
| | Glycérine | 5-30 | 5-30 |
| | Gélifiants de phase aqueuse | 0,1-1 | 0,1-1 |
| B | Alcool gras | 0,5-1,5 | 0,5-1,5 |
| | Beurre végétal | 1-5 | 1-5 |
| | Emollient | 5-20 | 5-20 |
| | Emulsionnant dérivé d'olive | 2-5 | - |
| | Cire émulsionnante | - | 2-5 |
| C | Agent de toucher | - | 1-3 |
| | Agent filmogène | - | 0,1-1 |
| | Ajusteur de pH | QSP | QSP |
| D | Purée végétale 1 | 30 | |
| | Purée végétale 1 | - | 30 |

Les compositions précitées ont été préparées selon le procédé suivant en utilisant pour les étapes de mélange : Rayneri /défloculeuse, comme contenant un bécher de 600 ml pour la réalisation de 400 gr de produit, une plaque chauffante, des spatules, des maryses et une balance.

Procédé :

Les matières premières de la phase A ont été pesées à l'aide de la balance et chauffées à 75°C en utilisant le Rayneri/Défloculeuse et la plaque chauffante.
Les ingrédients de la phase B ont été pesés à l'aide de la balance et chauffés à 75°C (bécher/plaque chauffante).
Lorsque les températures des phases A et B ont été atteintes, la phase B a été ajoutée dans la phase A sous Rayneri afin de réaliser une émulsion et la vitesse a été augmentée progressivement à 3000 tr/min. le mélange a été laissé émulsionner à cette vitesse pendant 3 min puis diminué à 800 tr/min. L'ensemble a été refroidi à une vitesse de 800 tr/min. Une fois la température de 45°C atteinte, les ingrédients de la phase C ont été ajoutés et la vitesse a été raugmentée à 1500 tr/min pendant 3 min. L'ensemble a été laissé refroidir à 30°C à 800 tr/min.
Une fois la base de crème réalisée et refroidie, la purée végétale a été ajoutée à froid, c'est-à-dire la base de crème a été diluée par l'ajout de la purée par simple agitation sans la nécessité de réchauffer l'ensemble à l'aide d'une plaque chauffante (phase D) en mélangeant 3 min à 1500 tr/min.
D'autres exemples de compositions cosmétiques ont été préparés à partir de la composition 2 précitée dans laquelle la concentration de purée végétale varie. Le tableau 8 ci-dessous résume les variations de concentration de la purée. Une étude de la stabilité et de l'homogénéité des compositions obtenues a été également effectuée. Cette étude a été réalisée par une évaluation visuelle de l'homogénéité du mélange de la base cosmétique et de la purée à tO après formulation. En particulier, une vérification de l'absence de déphasage, de l'homogénéité de la purée dans le support de formulation, de l'aspect visuel et sensoriel de la finesse de granulométrie adéquate pour une application non rincée par étalement d'une noix de produit sur le dos de la main jusqu'à pénétration et évaluation du film résiduel et vérification de l'absence de grains a été réalisée.
Par ailleurs, une vérification de la stabilité de la composition obtenue a été également réalisée par selon le procédé suivant. Chaque essai a été conditionné dans 4 piluliers de 50 ml qui ont été stockés à 4 températures différentes (4°C, TA, 40°C et 50°C). La stabilité du produit stocké à 4°C, TA et 40°C a été évaluée pendant 3 mois. Celle des produits stockés à 50°C a été évaluée pendant 1 mois. Un contrôle des piluliers stockés à 50°C a été réalisé toutes les semaines pendant un mois. Un contrôle des piluliers stockés à 4°C, TA et 40°C a été réalisé toutes les semaines pendant un mois puis tous les 15 jours jusqu'à 3 mois de stabilité. Lors de ces contrôles, l'aspect/odeur/couleur/l'homogénéité du produit ont été évalués. Le pilulier stocké à 4°C constituait le pilulier de référence.

Tableau 13 : exemples de composition cosmétique comprenant des purées végétales :

| compositions | 3 | 4 | 5 | 6 |
|---|---|---|---|---|
| % en purée | 25 | 30 | 40 | 50 |
| Résultats | émulsion fine, stable et homogène | émulsion fine, stable et homogène | émulsion fine, stable et homogène | émulsion fine, stable et homogène |

Les caractéristiques organoleptiques, microbiologiques et physico-chimiques de la purée obtenue à l'exemple 1 permettent à cette dernière d'être incorporée à fort pourcentage dans un produit cosmétique rincé ou non rincé et donc son utilisation cosmétique.

**Exemple 3: Test d'usage de deux exemples de compositions cosmétiques et évaluation des qualités cosmétiques de cette composition**

Dans cet exemple, les essais sont effectués avec une purée végétale correspondant à la purée obtenue dans l'exemple 1 précitée et avec la composition 2 de l'exemple 2 ci-dessus.

Un dosage biochimique de la purée végétale est effectué afin d'identifier et de quantifier notamment, les sucres totaux, les acides aminés totaux, les polyphénols, la vitamine C, les caroténoïdes, les anthocyanes, le glucose/fructose/saccharose et le pouvoir antioxydant (ORAC) de la purée.

Pour ce faire, la purée a été analysée selon les procédés décrits dans l'exemple 1.

Une évaluation des effets biologiques de la purée végétale est effectuée par :

1- une analyse transcriptomique comprenant un criblage large sur différents gènes pour voir s'il y a augmentation ou diminution de l'expression des gènes en présence de la purée et l'identification de marqueurs, et

2- la mesure d'effets biologiques ciblés en fonction des résultats de screening, par des méthodes in-vitro ou ex-vivo via, notamment la mesure de la prolifération kératinocytaire, l'étude de l'effet hydratant de la composition.

Une évaluation des effets d'un exemple de composition comprenant une purée végétale est également effectuée par :

3- un test d'usage et d'efficacité sur volontaires sains par application biquotidienne pendant 1 mois sur la peau de la composition par comparaison de la composition contenant 30% de purée végétale (composition 4 de l'exemple 2 ci-dessus) vs une composition placebo c'est-à-dire la même composition cosmétique sans purée, et

4- un test d'usage et d'efficacité sur volontaires sains par application biquotidienne pendant 1 mois de la composition via la comparaison de la composition contenant 30% de purée végétale, composition 4 de l'exemple 2 ci-dessus vs crème contenant un extrait aqueux (procédé d'obtention classique) obtenu avec mêmes lots de végétaux.

**Exemple 4 : Exemple d'obtention de purées végétales**

Dans le présent exemple, des purées ont été obtenues à partir de de roses, de pivoine et de framboises. En particulier des purées de roses et framboises, et de pivoines et framboises ont été obtenues.

Les purées de purées de roses/framboises ont été obtenues selon le procédé suivant. Pour chaque batch 10kg de fleurs et 10kg de fruits préalablement décongelés stockées à 4°C depuis 12h00, ont été broyés avec un broyeur de marque Stephan environ 10 minutes en chauffant à une température comprise entre 10 et 15°C.

L'ensemble roses/framboise a ensuite été raffiné par une raffineuse horizontale de marque Rossi Catelli. Une étape d'affinage a été rajoutée par rapport au procédé décrit dans l'exemple 1, afin d'optimiser la réduction dimensionnelle des végétaux. Un broyeur colloïdal de type broyeur à denture modèle MZ de chez FrymaKoruma a été utilisé pour cette étape. La purée a ensuite été homogénéisée selon le procédé décrit dans l'exemple 1 et traitée thermiquement :

- soit par pasteurisation selon le même barème que décrit dans l'exemple 1 (procédé 1),
- soit traité sur ligne pilote à chauffage ohmique d'une capacité de 200 kg/h et d'une puissance de 2X 30 kW (procédé 2)

Les purées de purées de pivoines-framboises ont été obtenues selon les 2 procédés suivants.

- Procédé 1 correspondant à celui utilisé pour l'obtention de purées de roses-framboises comprenant avec un traitement thermique par pasteurisation tel que décrit ci-dessus,
- Procédé 3 correspondant au procédé précité dans lequel le broyage au Stéphan a été réalisé pendant 25 min et ne comprenant pas l'étape de raffinage après le broyeur colloïdal. Les purées ont été traitées thermiquement par pasteurisation.

Ainsi 5 purées ont été obtenues pour lesquels la granulométrie, la teneur en matière sèche, en sucres totaux, en acide aminés libres, en polyphénols totaux et en protéines totales ont été mesurées. Ces caractéristiques ont été déterminées selon les procédés décrits dans l'exemple 1 ci-dessus. Pour la teneur en protéine totale, le procédé utilisé était la méthode de Bradford. Le tableau 14 ci-dessous résume les résultats obtenus.

Les caractéristiques des purées obtenues ont été comparées avec les caractéristiques d'un extrait classique, ce dernier étant obtenu classiquement par mise en contact d'un végétal (frais, sec, frais congelé...) à un taux de 5 à 25% avec un support d'extraction (eau, éthanol, glycérine, glycols, corps gras...ou mélange de ces derniers) avec un temps de contact entre 30 minutes et plusieurs jours, une température de mélange entre 10°C et 200°C, puis ont été classiquement réalisées des étapes de filtration jusqu'à la filtration stérilisante. Les caractéristiques d'un extrait classique, à savoir, en moyenne, les teneurs en matière sèche, sucres totaux, acides aminés, polyphénols totaux et protéines totales décrites dans le tableau 14 ci-dessous.

Tableau 14 caractéristique des purées obtenues

| Purée fleurs/ fruits | Granulométrie ($\mu$m) | Matière sèche (g/100g) | Sucres totaux (g/100g) | Acides aminés libres | Polyphénols totaux en équivalent acide gallique (mg/100g) | Protéines totales |
|---|---|---|---|---|---|---|
| 1 Rose - framboise (non traité) | 85 | 11,46% | 4,17% | 0,32% | 0,48% | - |
| 2 Rose - framboise (process 1) | 85 | 11,44% | 4,21% | 0,32% | 0,32% | - |
| 3 Rose - framboise (process 2) | 85 | 11,40% | 4,02% | 0,33% | 0,33% | - |
| 4 Pivoine - framboise (process1) | 106 | 18,35% | 5,92% | 0,17% | 1,04 | 0,45% |
| 5 Pivoine - framboise (process3) | 109 | 15,95% | 5,43% | 0,17% | 0,68 | 0,34% |
| Extrait du commerce | - | 1-3% | 0,95% | 0,06% | 0,19 | - |

En outre, la teneur en vitamine C a été mesurée dans les purées 1 et 2 de Rose - framboise selon les procédés décrits ci-dessus et était respectivement égal à 112mg/kg et 91 mg/kg de purée.

Tel que démontré dans cet exemple, les purées selon l'invention, notamment les purées obtenues à partir d'un mélange fleurs - fruits, comprennent des teneurs en principes actifs cosmétiques au moins trois fois supérieurs à l'extrait disponible dans le commerce.

En outre, cet exemple démontre clairement que la purée selon l'invention a une teneur en matière sèche de 5 à 10 fois supérieure au extrait connue tout en ayant une granulométrie permettant, notamment, avantageusement une utilisation cosmétique ne nécessitant pas d'étape de rinçage après application.

**Revendications**

1. Utilisation cosmétique d'une purée végétale, ladite purée ayant une granulométrie moyenne de 50 $\mu$m à 2 cm et dans laquelle ladite purée comprend une teneur en matière sèche de 5 à 40 % en poids par rapport au poids total de la purée.

2. Utilisation selon la revendication 1, dans laquelle ladite purée est à un pH de 1 à 6.

3. Utilisation selon l'une quelconque des revendications précédentes dans laquelle ladite purée est obtenue à partir d'une matière végétale fraiche ou surgelée.

4. Utilisation selon l'une quelconque des revendications précédentes, dans laquelle la purée est obtenue par un procédé comprenant les étapes suivantes :

a) broyage du végétal,
b) traitement microbiologique choisi parmi un traitement thermique du broyat obtenu à l'étape a) à une température de 20 à 150°C, un traitement haute pression à une pression de 2000 à 5000 bars, par un traitement par ionisation, un traitement par application d'hautes fréquences, un traitement par application d'un courant ohmique, un traitement par pasteurisation en autoclave, ou par des procédés de stérilisation , de préférence UHT, les techniques d'infusion ou d'injection directe de vapeur dans le broyat obtenu à l'étape a) ou un mélange de ceux-ci

5. Utilisation selon l'une quelconque des revendications précédentes, dans laquelle ladite purée est une purée végétale choisie parmi une purée de fruits, une purée de fleurs ou un mélange de celles-ci.

6. Utilisation selon l'une quelconque des revendications précédentes, dans laquelle ladite purée est une purée du totum du végétal.

7. Utilisation selon l'une quelconque des revendications précédentes, dans laquelle ladite purée est une purée d'un végétal choisi dans le groupe comprenant les pivoines, les rosiers, le bigaradier, les framboisiers, les abricotiers, le jasmin, la violette, le safran, le chèvrefeuille, le lys, la verveine, l'argousier, la pêche, la mirabelle, les agrumes, la griotte, la cerise, la pomme, le cassis, la canneberge, le kiwi, la poire, la groseille, la fraise, la grenade, la myrtille, la mure, le sorbe, l'églantier, l'acérola, la carotte, la prune, le melon, la goyave, la mangue, le potiron ou un mélange de ceux-ci.

8. Purée végétale cosmétique susceptible d'être obtenue par le procédé défini dans l'une quelconque des revendications 3 à 7.

9. Procédé de traitement cosmétique non thérapeutique comprenant une étape d'application sur la peau et/ou les phanères d'une purée végétale selon l'une quelconque des revendications 1 à 8.

10. Procédé de traitement cosmétique non thérapeutique comprenant une étape d'application sur la peau d'une purée végétale selon l'une quelconque des revendications 1 à 8, puis éventuellement une étape de rinçage.

11. Procédé de traitement cosmétique non thérapeutique des cheveux consistant à appliquer sur les cheveux une purée végétale selon l'une quelconque des revendications 1 à 8, puis éventuellement à effectuer un rinçage.

Europäisches
Patentamt

European
Patent Office

Office européen
des brevets

**RAPPORT DE RECHERCHE EUROPEENNE**

Numéro de la demande

EP 16 18 1341

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2003/008058 A1 (ARIGA TOYOHIKO [JP] ET AL) 9 janvier 2003 (2003-01-09) | 8 | INV. A61Q5/00 |
| Y | * figure 1 * * alinéa [0002] - alinéa [0003] * * alinéa [0018] - alinéa [0023] * * alinéa [0030] - alinéa [0041] * * exemple 6 * * alinéa [0079] - alinéa [0080] * * alinéa [0086] * * revendications 1,5,10 * | 1-11 | A61Q19/00 A61Q19/08 A61K8/97 |
| Y | US 2005/079236 A1 (AHRENS JASON ROBERT [US]) 14 avril 2005 (2005-04-14) * alinéa [0002] * * alinéa [0007] * * alinéa [0008] - alinéa [0011] * * alinéa [0016] * | 1-11 | |
| A | WO 99/48456 A1 (UNILEVER PLC [GB]; UNILEVER NV [NL]; LEVER HINDUSTAN LTD [IN]) 30 septembre 1999 (1999-09-30) * page 5, ligne 15 - page 10, ligne 17 * * tableau I to IV * | 1-11 | |
| X | EP 0 777 975 A1 (NESTLE SA [CH]) 11 juin 1997 (1997-06-11) * exemple 1 * | 8 | |
| X | EP 1 135 994 A2 (PASELUMA ELETTRICA S R L [IT]) 26 septembre 2001 (2001-09-26) * alinéa [0018] - alinéa [0020] * * alinéa [0027] * * revendications 1,3 * | 8 | |
| X | US 2003/091694 A1 (REMO ZUCCATO [IT]) 15 mai 2003 (2003-05-15) * exemples * | 8 | |

DOMAINES TECHNIQUES RECHERCHES (IPC)

A61Q
A61K

-----

-----

-----

-----

-----

-/--

Le présent rapport a été établi pour toutes les revendications

| Lieu de la recherche | Date d'achèvement de la recherche | Examinateur |
|---|---|---|
| Munich | 7 septembre 2016 | Irwin, Lucy |

Europäisches
Patentamt

European
Patent Office

Office européen
des brevets

# RAPPORT DE RECHERCHE EUROPEENNE

Numéro de la demande

EP 16 18 1341

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | WO 2012/159919 A1 (UNILEVER PLC [GB]; UNILEVER NV [NL]; UNILEVER HINDUSTAN [IN]; BATCHELO) 29 novembre 2012 (2012-11-29) <br> * page 14, ligne 6 - page 15, ligne 13 * <br> * revendications 1,2 * <br> ----- | 11 | |
| A | US 2007/122509 A1 (CHOMCZYNSKI PIOTR [US]) 31 mai 2007 (2007-05-31) <br> * le document en entier * <br> ----- | 1-11 | |

**DOMAINES TECHNIQUES RECHERCHES (IPC)**

Le présent rapport a été établi pour toutes les revendications

| Lieu de la recherche | Date d'achèvement de la recherche | Examinateur |
|---|---|---|
| Munich | 7 septembre 2016 | Irwin, Lucy |

CATEGORIE DES DOCUMENTS CITES

X : particulièrement pertinent à lui seul
Y : particulièrement pertinent en combinaison avec un
  autre document de la même catégorie
A : arrière-plan technologique
O : divulgation non-écrite
P : document intercalaire

T : théorie ou principe à la base de l'invention
E : document de brevet antérieur, mais publié à la
  date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 16 18 1341

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

07-09-2016

| Document brevet cité au rapport de recherche | | Date de publication | Membre(s) de la famille de brevet(s) | | Date de publication |
|---|---|---|---|---|---|
| US 2003008058 | A1 | 09-01-2003 | JP | 2003070444 A | 11-03-2003 |
| | | | US | 2003008058 A1 | 09-01-2003 |
| US 2005079236 | A1 | 14-04-2005 | AUCUN | | |
| WO 9948456 | A1 | 30-09-1999 | AR | 017986 A1 | 24-10-2001 |
| | | | AU | 736879 B2 | 02-08-2001 |
| | | | AU | 3143699 A | 18-10-1999 |
| | | | BR | 9909055 A | 05-12-2000 |
| | | | CA | 2319469 A1 | 30-09-1999 |
| | | | CN | 1293557 A | 02-05-2001 |
| | | | CZ | 20003521 A3 | 16-05-2001 |
| | | | DE | 69910185 D1 | 11-09-2003 |
| | | | DE | 69910185 T2 | 26-02-2004 |
| | | | EP | 1066009 A1 | 10-01-2001 |
| | | | ES | 2204113 T3 | 16-04-2004 |
| | | | HK | 1032734 A1 | 21-01-2004 |
| | | | ID | 26039 A | 16-11-2000 |
| | | | JP | 2002507556 A | 12-03-2002 |
| | | | PL | 343074 A1 | 30-07-2001 |
| | | | RU | 2207108 C2 | 27-06-2003 |
| | | | TW | 581673 B | 01-04-2004 |
| | | | US | 6159487 A | 12-12-2000 |
| | | | WO | 9948456 A1 | 30-09-1999 |
| | | | ZA | 9900526 B | 25-07-2000 |
| EP 0777975 | A1 | 11-06-1997 | AR | 004892 A1 | 10-03-1999 |
| | | | AT | 238692 T | 15-05-2003 |
| | | | AU | 718456 B2 | 13-04-2000 |
| | | | AU | 7419096 A | 12-06-1997 |
| | | | BR | 9605861 A | 25-08-1998 |
| | | | CA | 2192378 A1 | 08-06-1997 |
| | | | CN | 1156554 A | 13-08-1997 |
| | | | CZ | 9603592 A3 | 11-06-1997 |
| | | | DE | 69530597 D1 | 05-06-2003 |
| | | | DE | 69530597 T2 | 13-11-2003 |
| | | | EP | 0777975 A1 | 11-06-1997 |
| | | | ES | 2198432 T3 | 01-02-2004 |
| | | | FI | 964874 A | 08-06-1997 |
| | | | HU | 9603369 A2 | 29-09-1997 |
| | | | IL | 119747 A | 28-10-1999 |
| | | | JP | H09173027 A | 08-07-1997 |
| | | | NO | 965216 A | 09-06-1997 |
| | | | NZ | 299874 A | 24-11-1997 |
| | | | PL | 317342 A1 | 09-06-1997 |
| | | | PT | 777975 E | 30-09-2003 |

EPO FORM P0460

Pour tout renseignement concernant cette annexe : voir Journal Officiel de l'Office européen des brevets, No.12/82

## ANNEXE AU RAPPORT DE RECHERCHE EUROPEENNE
## RELATIF A LA DEMANDE DE BREVET EUROPEEN NO.

EP 16 18 1341

La présente annexe indique les membres de la famille de brevets relatifs aux documents brevets cités dans le rapport de recherche européenne visé ci-dessus.

Lesdits members sont contenus au fichier informatique de l'Office européen des brevets à la date du

Les renseignements fournis sont donnés à titre indicatif et n'engagent pas la responsabilité de l'Office européen des brevets.

07-09-2016

| Document brevet cité au rapport de recherche | | | Date de publication | Membre(s) de la famille de brevet(s) | | | Date de publication |
|---|---|---|---|---|---|---|---|
| | | | | US | 5922374 | A | 13-07-1999 |
| | | | | ZA | 9610249 | B | 05-06-1998 |
| EP 1135994 | | A2 | 26-09-2001 | AT | 327684 | T | 15-06-2006 |
| | | | | AU | 784739 | B2 | 08-06-2006 |
| | | | | AU | 2982801 | A | 27-09-2001 |
| | | | | CA | 2341788 | A1 | 22-09-2001 |
| | | | | CN | 1326685 | A | 19-12-2001 |
| | | | | DE | 60120018 | T2 | 28-12-2006 |
| | | | | DK | 1135994 | T3 | 18-09-2006 |
| | | | | EP | 1135994 | A2 | 26-09-2001 |
| | | | | ES | 2267619 | T3 | 16-03-2007 |
| | | | | IT | VI20000049 | A1 | 24-09-2001 |
| | | | | JP | 2001258470 | A | 25-09-2001 |
| | | | | PT | 1135994 | E | 31-10-2006 |
| | | | | US | 2001024669 | A1 | 27-09-2001 |
| US 2003091694 | | A1 | 15-05-2003 | US | 2003091694 | A1 | 15-05-2003 |
| | | | | US | 2005153017 | A1 | 14-07-2005 |
| WO 2012159919 | | A1 | 29-11-2012 | AUCUN | | | |
| US 2007122509 | | A1 | 31-05-2007 | AU | 2007324036 | A1 | 29-05-2008 |
| | | | | CA | 2668471 | A1 | 29-05-2008 |
| | | | | CN | 101616604 | A | 30-12-2009 |
| | | | | EP | 2079320 | A2 | 22-07-2009 |
| | | | | JP | 2010509352 | A | 25-03-2010 |
| | | | | KR | 20090081427 | A | 28-07-2009 |
| | | | | US | 2007122509 | A1 | 31-05-2007 |
| | | | | WO | 2008063857 | A2 | 29-05-2008 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **LYKKESFELDT et al.** *Anal Biochem.,* 15 Juin 2000, vol. 282 (1), 89-93

- **GEORGÉ.** *J Agric Food Chem.,* 09 Mars 2005, vol. 53 (5), 1370-3